# EUROPÄISCHE PATENTSCHRIFT

(11) **EP 2 566 533 B1**
(45) Veröffentlichungstag und Bekanntmachung des Hinweises auf die Patenterteilung: **26.11.2014**
(21) Anmeldenummer: 11758080.3
(22) Anmeldetag: 29.04.2011
(51) Int. Cl.: A61M 1/10, F04D 13/06, F04D 29/58

(54) **BLUTPUMPE MIT EINEM ROTOR**
BLOOD PUMP COMPRISING A ROTOR
POMPE À SANG POURVUE D'UN ROTOR

(30) Priorität: 22.06.2010 DE 102010024650; 04.05.2010 DE 102010019403
(43) Veröffentlichungstag der Anmeldung: 13.03.2013
(73) Patentinhaber: MEDOS Medizintechnik AG, 52222 Stolberg (DE)
(72) Erfinder: BAUMGARTNER, Robert, 52146 Würselen (DE); HENSELER, Andreas, 52152 Simmerath (DE); MATTERN, Benjamin, 52062 Aachen (DE)
(74) Vertreter: Patentanwaltskanzlei Liermann-Castell
(86) Internationale Anmeldenummer: PCT/DE2011/001004
(87) Internationale Veröffentlichungsnummer: WO 2012/006976

(56) Entgegenhaltungen:
- EP-A1- 2 127 691
- WO-A1-2005/028000
- WO-A2-2007/084339
- DE-A1-102006 036 948
- GB-A- 1 383 811
- US-A- 4 643 641
- US-A- 4 984 972

## Beschreibung

Die Erfindung betrifft eine Blutpumpe mit einem Rotor, der über ein Lager in einem Pumpengehäuse gelagert ist.

Derartige Blutpumpen sind in verschiedenen Ausführungsformen bekannt. Zunächst wurden Radial- und Axialpumpen unterschieden. Besonders gute Ergebnisse wurden mit einer diagonalen Strömung erzielt.

Die Erfindung betrifft daher insbesondere das Gebiet der Diagonalpumpen, siehe z. B. DE 102006036948.

Ein wesentlicher Einsatzbereich derartiger Blutpumpen ist die Förderung von menschlichem Blut im Rahmen extrakoroporaler Kreisläufe. Die Blutpumpe übernimmt dann vollständig oder teilweise die Pumpfunktion des Herzens und sie kann zur Herzunterstützung, zur Regeneration oder Überbrückung für einen Zeitraum von mehreren Tagen eingesetzt werden. Der Einsatz erfolgt im Zusammenhang mit den klinisch üblichen Komponenten wie PVC-Schlauchsystem, Oxygenatoren, arteriellen Filtern und bei Bedarf diversen Reservoiren. An Stelle eines PVC-Schlauchsystems können auch Silikonschlauchstücke bis hin zu ganzen Silikonschlauchsystemen eingesetzt werden.

Zur Steuerung, Energieversorgung und Überwachung dienen spezielle Konsolen, die auch den Antrieb übernehmen können.

Vorteilhaft ist es bei derartigen Pumpen, wenn das Prinzip der Magnetkupplung eingesetzt wird, um den Blutteil von dem Antriebsteil zu entkoppeln. Dies ermöglicht es, die blutführenden Oberflächen sehr klein zu halten. Eine kompakte Bauweise ermöglicht einen patientenschonenden und patientennahen Einsatz.

Bei derartigen Blutpumpen wird der Rotor vorzugsweise auf einer Lagerkugel aus Keramik oder Aluminiumoxid gelagert. Im Bereich der Lagerung wirken Kräfte, die zu einer Erwärmung oder Schädigung der Pumpe führen können.

Der Erfindung liegt daher die Aufgabe zugrunde, eine derartige Blutpumpe weiterzuentwickeln.

Diese Aufgabe wird mit einer gattungsgemäßen Blutpumpe wie beansprucht gelöst, bei der das Gehäuse einen von seiner äußeren Wandung zur Gehäuseinnenseite weisenden Stift aus Metall aufweist.

Ein derartiger Stift aus einem stabilen und wärmeleitfähigen Material ermöglicht es einerseits, Wärme durch die Gehäusewandung abzuleiten und andererseits, vom Gehäuse nach innen ragende Gehäuseteile zu stabilisieren. Die Blutpumpe braucht dabei weder eine Welle noch dynamische Dichtungen.

Besonders vorteilhaft ist es, dass der Stift kegelförmig ausgebildet ist. Er erlaubt es somit, von einem speziellen Punkt innerhalb des Gehäuses Wärme aufzunehmen und zur Gehäuseaußenseite abzuleiten. Außerdem ermöglicht eine kegelförmige Ausbildung des Stiftes eine besondere mechanische Stabilität.

Um zu verhindern, dass der Stift mit dem im Gehäuse geführten Blut in Berührung kommt, ist der Stift in einer kegelförmigen Gehäuseausformung angeordnet. Der Stift kann somit aus dem Gehäuseinneren und insbesondere aus dem Lagerbereich Wärme abführen, ohne mit dem Blut selber in Berührung zu kommen. Besonders vorteilhaft ist es hierbei, wenn der Stift bis nahe an das Lager herangeführt wird, um aus dem Lagerbereich Wärme abzuführen und den Lagerbereich zu stabilisieren.

Um das Lager möglichst nah am Bluteinlass zu positionieren, wird vorgeschlagen, dass die kegelförmige Gehäuseausformung sich innerhalb der Schaufeln und vorzugsweise über die gesamte Länge der Schaufeln erstreckt. Dadurch wird erzielt, dass die Schaufeln des Rotors radial außerhalb des kegelförmigen Stiftes und radial außerhalb der kegelförmigen Gehäuseausformung angeordnet sind. Somit wird auch zwischen stationärer kegelförmiger Gehäuseausformung und dem Rotor entstehende Wärme durch den Metallstift abgeleitet.

Der Rotor kann kontinuierlich oder intermittierend drehen. Insbesondere für ein schnelles Beschleunigen des Rotors wird vorgeschlagen, dass der Durchmesser des Rotors kleiner als 30 mm, vorzugsweise kleiner als 28 mm ist. Der geringe Durchmesser reduziert die Trägheitskräfte und erleichtert dadurch die Beschleunigung. Somit kann der Rotor auch zum pulsatilen Einsatz ideal verwendet werden. Im pulsatilen Betrieb wird eine Drehzahl von 100 1/min bis 2500 1/min in 100er Schritten vorgeschlagen. Die Frequenz liegt dann bei 40 bis 90 1/min.

Insbesondere in Verbindung mit dem geringen Durchmesser ist es vorteilhaft, wenn das Gewicht des Rotors möglichst klein gehalten wird. Dadurch werden die Massenträgheitskräfte reduziert, um die Beschleunigung zu erleichtern. Bei einer vorteilhaften Ausführungsvariante hat der Rotor ein Gewicht von weniger als 10 g, vorzugsweise sogar weniger als 8,5 g.

In entsprechender Weise kann der Durchmesser der Rotordeckscheibe kleiner als 32 mm, vorzugsweise kleiner als 30 mm sein.

Die spezielle Ausbildung des Rotors und der Deckscheibe ist auch unabhängig von den übrigen Merkmalen der Erfindung erfindungswesentlich.

Ein weiteres, auch unabhängig von den übrigen Merkmalen erfindungswesentliches Merkmal liegt in der Ausbildung des Gehäuses. Um auf einfache Art und Weise die Antriebseinheit vom "Blutteil" zu trennen, wird vorgeschlagen, dass das Gehäuse eine radial äußere Gehäusewandung und eine sich in diese hinein erstreckende Motoraufnahme aufweist. Der Motor kann dadurch leicht vom Gehäuse getrennt werden und das Gehäuse wird gleichzeitig als Motorhalterung verwendet.

Eine besonders kompakte Bauweise wird dadurch erzielt, dass zwischen radial äußerer Gehäusewandung und der Motoraufnahme ein Ringspalt ausgebildet ist. In diesem Ringspalt kann Blut geführt werden, das vom Rotor zum Blutauslass fließt. Durch die Entkopplung von Blutteil und Antriebsteil wird es möglich, die Antriebseinheit mehrfach zu verwenden und die Blut führende Oberfläche sehr klein zu halten. Das Pumpenmaterial wird dabei so gewählt, dass es mit einer Blutgerinnung hemmenden Beschichtung wie beispielsweise Reoparin oder Bioline beschichtet werden kann.

Vorteilhaft ist es, wenn der Rotor mindestens einen Kupplungsmagnet aufweist, um ein Drehmoment von einem Motor berührungslos auf den Rotor zu übertragen. Dazu können im Rotor angeordnete Segmentmagnete oder ein Ringmagnet verwendet werden. Die Verwendung eines Ringmagneten ist auch unabhängig von den zuvor genannten Merkmalen erfindungswesentlich. Hierbei hat der Kupplungsmagnet in einem bevorzugten Ausführungsbeispiel einen magnetischen Rückschluss. Sofern die Vorderseite des Rotors dem Motor zugewandt ist, ist der Rückschluss an der Rückseite des Rotors angeordnet. Der Rückschluss verbindet die Segmentmagnete und sorgt dafür, dass zwischen Rotor und Antriebsmagneten eine magnetische Feldstärke entstehen kann, mit der eine Kraft von über 20 N übertragen werden kann.

Weiterhin ist es von Vorteil, wenn der Rotor eine Spühlbohrung aufweist, die zum Lager führt.

Am Lager entsteht eine hohe Reibung mit der Folge einer großen Wärmeentwicklung. Diese Wärme kann wie zuvor beschrieben durch einen gut Wärme leitenden Stift abgeleitet werden, der vorzugsweise aus Stahl hergestellt ist. Alternativ oder kumulativ wird vorgeschlagen, dass der Rotor über ein Lager mit einem in einer Lagerschale liegenden Kugelsegment im Pumpengehäuse gelagert ist. Dabei sollte das Kugelsegment maximal den Radius der Lagerschale haben, um sicher im Lager zu liegen. Vorzugsweise ist der Radius des Kugelsegments kleiner als der Radius der Lagerschale, so dass ein Punktlager entsteht. Dabei kann das Kugelsegment ein Teil einer Kugel sein. Die Ausbildung des Lagers ist auch unabhängig von den zuvor genannten Merkmalen erfindungswesentlich.

Das Kugelsegment kann dabei am Stift und am Rotor ausgebildet sein. Es hat sich herausgestellt, dass es vorteilhaft ist, wenn der Rotor das Kugelsegment aufweist.

Damit möglichst wenig Abrieb entsteht und um eine gute Wärmeableitung zu gewährleisten, wird vorgeschlagen, dass ein Teil des Lagers aus PTFE, Stahl, Keramik oder Glas, vorzugsweise Borosilikatglas hergestellt ist. Dieser Teil ist vorzugsweise das Kugelsegment oder die Kugel, während der Stift vorzugweise aus Stahl hergestellt ist. Die Auswahl der Materialien ist auch unabhängig von den zuvor genannten Merkmalen erfindungswesentlich.

Da im Gehäuse vor allem im Bereich der Lagerung Temperaturen von über 200 °C auftreten können wird vorgeschlagen, dass mindestens ein Teil des Gehäuses aus einem Polyetherketon hergestellt ist. Dies sind hochtemperaturbeständige thermoplastische Kunststoffe, wie beispielsweise Polyetheretherketon (PEEK). Dieses Merkmal ist auch unabhängig von den zuvor genannten Merkmalen erfindungswesentlich.

Ein besonderer konstruktiver Aufbau ermöglicht es, eine derartige Blutpumpe herzustellen, bei der das Primingvolumen unter 17 ml, vorzugsweise unter 15 ml ist.

Ein Ausführungsbeispiel einer erfindungsgemäßen Blutpumpe ist in der Zeichnung dargestellt und wird im Folgenden näher erläutert. Es zeigt
Figur 1 eine Seitenansicht der Blutpumpe
Figur 2 eine Draufsicht auf die in Figur 1 gezeigte Blutpumpe ,
Figur 3 einen Schnitt durch die in Figur 1 gezeigte Blutpumpe längs der Linie C-C.
Figur 4 eine Seitenansicht einer alternativen Ausführungsform der Blutpumpe
Figur 5 eine Draufsicht auf die in Figur 4 gezeigte Blutpumpe und
Figur 6 einen Schnitt durch die in Figur 4 gezeigte Blutpumpe längs der Linie F-F.

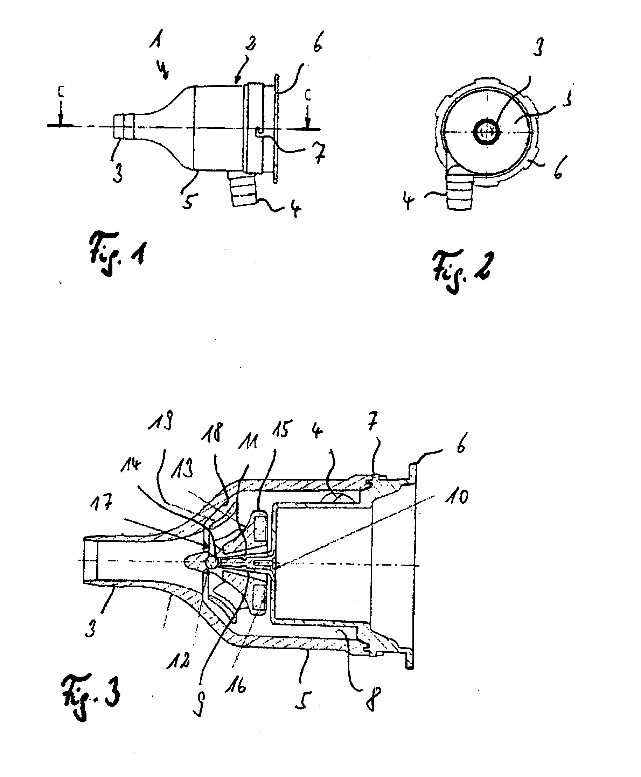

Die in Figur 1 gezeigte Blutpumpe 1 weist ein Pumpengehäuse 2 mit einem Zulauf 3 und einem Ablauf 4 auf.

Das Pumpengehäuse 2 besteht aus einer radial äußeren Gehäusewandung 5 und einer sich in diese hinein erstreckende Motoraufnahme 6. Die Motoraufnahme 6 ist formschlüssig über einen Verschluss 7 mit der äußeren Gehäusewandung 5 verbunden.

Die Motoraufnahme 6 ragt derart in die Gehäusewandung 5 hinein, dass sich zwischen der Gehäusewandung 5 und der Motoraufnahme 6 ein Ringspalt 8 ergibt. Koaxial zum Zulauf 3 ist an der Motoraufnahme 6 eine kegelförmige Gehäuseausformung 9 ausgebildet, in die sich kegelförmig ein Stift 10 erstreckt. Der Stift 10 ist über einen Einschnitt 11 fest mit der kegelförmigen Gehäuseausformung 9 verbunden.

An der Spitze der kegelförmigen Gehäuseausformung 9 befindet sich eine Lagerkugel 12, die fest mit einem Rotor 13 verklebt ist. Diese Lagerkugel 12 läuft in einer Kalotten-Form der kegelförmigen Gehäuseausformung 9 mit dem darin angeordneten Stift 10. Dadurch ist der Rotor 13 mittels des Lagers 14 im Pumpengehäuse 2 gelagert.

Der Rotor 13 weist Schaufeln 15 auf, an denen Kupplungsmagnete 16 befestigt sind, um das Drehmoment eines Motors (nicht gezeigt) berührungslos auf den Rotor 13 zu übertragen. Die Kupplungsmagnete 16 im Rotor 13 können als einzelne Viertelsegmentmagnete ausgeführt sein. Vorteilhaft ist ein Ringmagnet.

Der Rotor 13 hat einen Durchmesser von etwa 26 mm und in der Nähe des Lagers 14 eine Spühlbohrung 17, über die Blut vom Einlass 3 zum Lager 14 fließt.

Zwischen dem Rotor 13 und der Gehäuseinnenseite 18 ist eine Rotordeckscheibe 19 angeordnet, die einen Durchmesser von ca. 28 mm hat.

Die Blutpumpe kann für einen Zeitraum von mindestens sieben Tagen eingesetzt werden. Die Anwendungsdauer reicht von bis zu 6 Stunden bis zu mehreren Wochen. In der Praxis hat sich bisher gezeigt, dass die Pumpe einem kontinuierlichen Einsatz von mehr als 50 Tagen ohne Weiteres Stand hält. Das diagonalförmige Laufrad und damit die diagonalförmige Flussrichtung vereinen die Vorteile von Zentrifugalpumpen und Axialpumpen. Die Pumpe erzeugt in einem Drehzahlbereich von 0 bis 10 000 U/min einen kontinuierlichen oder einen pulsatilen Volumenstrom von 0 bis 8 Liter pro Minute bei einer maximalen Druckdifferenz von bis zu 650 mmHg. Der maximale Förderdruck liegt in der Regel etwas unter 600 mmHg.

Der steril und pyrogenfrei ausgelieferte Pumpenkopf ist mindestens drei Jahre lagerungsfähig und vor Transportschäden ausreichend geschützt.

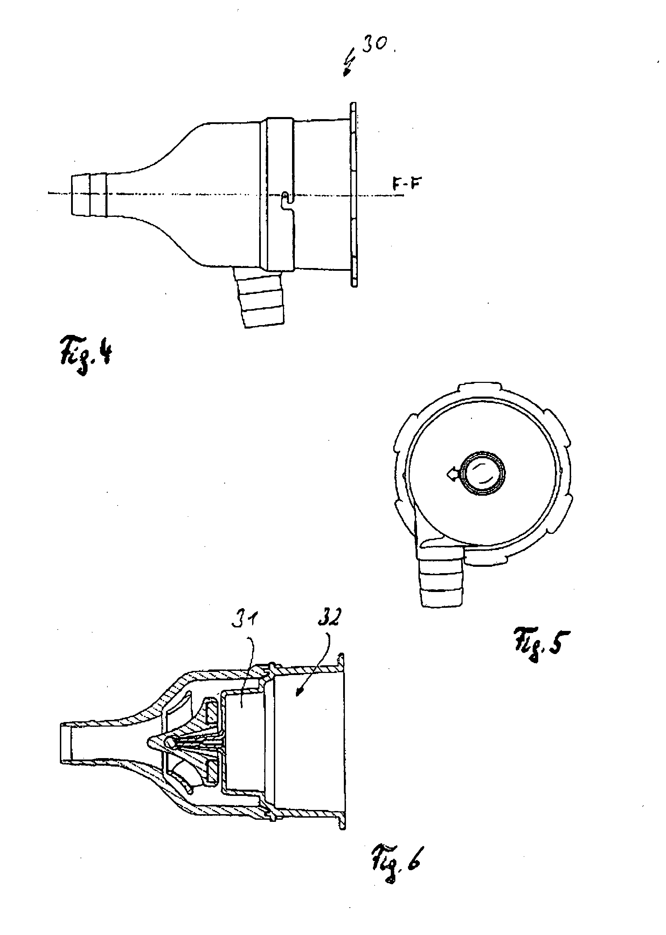

Die Figuren 4 bis 6 zeigen eine leicht abgewandelte Ausführungsform einer Blutpumpe 30. Während die in den Figuren 1 bis 3 gezeigte Ausführungsform ein Primingvolumen von 16 ml hat, liegt das Primingvolumen bei dieser abgewandelten Ausführungsform bei unter 15 ml. Dies wird durch einen verkürzten in die Pumpe hineinragenden Teil 31 der Motoraufnahme 32 erreicht.

## Patentansprüche

1. Blutpumpe (1) mit einem Rotor (13), der über ein Lager (14) in einem Pumpengehäuse (2) gelagert ist, wobei das Pumpengehäuse (2) einen von seiner Außenwandung (5) zur Gehäuseinnenseite (8) hin weisenden Stift (10) aus Metall aufweist, ***dadurch gekennzeichnet, dass*** der Stift (10) kegelförmig ausgebildet ist, in einer kegelförmigen Gehäuseausformung (9) angeordnet ist und an einer Außenseite der Außenwandung (5) des Pumpengehäuses (2) derart angeordnet ist, dass er erlautet Wärme zur Gehäuseaußenseite abzuleiten.

2. Blutpumpe nach Anspruch 1, ***dadurch gekennzeichnet, dass*** die kegelförmige Gehäuseausformung (9) sich innerhalb der Schaufeln (15) und vorzugsweise über die gesamte Länge der Schaufeln (15) erstreckt.

3. Blutpumpe nach einem der vorhergehenden Ansprüche, ***dadurch gekennzeichnet, dass*** der Durchmesser des Rotors kleiner als 30 mm, vorzugsweise kleiner als 28 mm ist.

4. Blutpumpe nach einem der vorhergehenden Ansprüche, ***dadurch gekennzeichnet, dass*** der Durchmesser einer Rotordeckscheibe (19) kleiner als 32 mm, vorzugsweise kleiner als 30 mm ist.

5. Blutpumpe nach einem der vorhergehenden Ansprüche, ***dadurch gekennzeichnet, dass*** der Rotor ein Gewicht von weniger als 10 g, vorzugsweise sogar weniger als 8,5 g aufweist.

6. Blutpumpe nach einem der vorhergehenden Ansprüche, ***dadurch gekennzeichnet, dass*** das Gehäuse eine radial äußere Gehäusewandung (5) und eine sich in diese hinein erstreckende Motoraufnahme (6) aufweist, wobei die Motoraufnahme (6) formschlüssig über einen Verschluss (7) mit der äußeren Gehäusewandung (5) verbunden ist und zwischen radial äußerer Gehäusewandung (5) und der Motoraufnahme (6) ein Ringspalt (8) ausgebildet ist.

7. Blutpumpe nach einem der vorhergehenden Ansprüche, ***dadurch gekennzeichnet, dass*** der Rotor (13) mindestens einen Kupplungsmagnet (16) aufweist, um ein Drehmoment von einem Motor berührungslos auf den Rotor (13) zu übertragen.

8. Blutpumpe nach einem der vorhergehenden Ansprüche, ***dadurch gekennzeichnet, dass*** der Rotor (13) eine Spühlbohrung (17) aufweist, die zum Lager (14) führt.

9. Blutpumpe insbesondere nach einem der vorhergehenden Ansprüche, ***dadurch gekennzeichnet, dass*** der Rotor (13) über ein Lager (14) mit einem in einer Lagerschale liegenden Kugelsegment im Pumpengehäuse (2) gelagert ist.

10. Blutpumpe nach Anspruch 9, ***dadurch gekennzeichnet, dass*** der Rotor das Kugelsegment aufweist.

11. Blutpumpe nach einem der vorhergehenden Ansprüche, ***dadurch gekennzeichnet, dass*** ein Teil des Lagers aus PTFE hergestellt ist.

12. Blutpumpe nach einem der vorhergehenden Ansprüche, ***dadurch gekennzeichnet, dass*** ein Teil des Lagers aus Edelstahl hergestellt ist.

13. Blutpumpe nach einem der vorhergehenden Ansprüche, ***dadurch gekennzeichnet, dass*** ein Teil des Lagers aus Keramik oder Glas, vorzugsweise Borosilikatglas hergestellt ist.

14. Blutpumpe nach einem der vorhergehenden Ansprüche, ***dadurch gekennzeichnet, dass*** mindestens ein Teil des Gehäuses aus einem Polyetherketon hergestellt ist.

15. Blutpumpe insbesondere nach einem der vorhergehenden Ansprüche, ***dadurch gekennzeichnet, dass*** das Primingvolumen der Blutpumpe unter 17 ml, vorzugsweise unter 15 ml ist.

## Claims

1. A blood pump (1) comprising a rotor (13), which is mounted via a bearing (14) in a pump housing (2), the pump housing (2) having a pin (10) made of metal pointing from the outer wall (5) of said pump housing to the housing inner side *(8), **characterized in that*** the pin (10) is conical, is arranged in a conical housing shaping (9) and is arranged on an outer side of the outer wall (5) of the pump housing (2) in such a way that said pin allows heat to be dissipated to the housing outer side.

2. The blood pump according to Claim 1, ***characterized in that*** the conical housing shaping (9) extends within the blades (15) and preferably over the entire length of the blades (15).

3. The blood pump according to one of the preceding claims, ***characterized in that*** the diameter of the rotor is smaller than 30 mm, preferably smaller than 28 mm.

4. The blood pump according to one of the preceding claims, ***characterized in that*** the diameter of a rotor cover disc (19) is smaller than 32 mm, preferably smaller than 30 mm.

5. The blood pump according to one of the preceding claims, ***characterized in that*** the rotor has a weight of less than 10 g, preferably even less than 8.5 g.

6. The blood pump according to one of the preceding claims, ***characterized in that*** the housing has a radially outer housing wall (5) and a motor mount (6) extending thereinto, the motor mount (6) being connected in a form-fitting manner via a closure (7) to the outer housing wall (5) and an annular gap (8) being formed between the radially outer housing wall (5) and the motor mount (6).

7. The blood pump according to one of the preceding claims, ***characterized in that*** the rotor (13) has at least one coupling magnet (16) in order to contactlessly transmit a torque from a motor to the rotor (13).

8. The blood pump according to one of the preceding claims, ***characterized in that*** the rotor (13) has a flushing bore (17), which leads to the bearing (14).

9. A blood pump in particular according to one of the preceding claims, ***characterized in that*** the rotor (13) is mounted in the pump housing (2) via a bearing (14) with a spherical segment arranged in a bearing shell.

10. The blood pump according to Claim 9, ***characterized in that*** the rotor comprises the spherical segment.

11. The blood pump according to one of the preceding claims, ***characterized in that*** part of the bearing is produced from PTFE.

12. The blood pump according to one of the preceding claims, ***characterized in that*** part of the bearing is produced from high-grade steel.

13. The blood pump according to one of the preceding claims, ***characterized in that*** part of the bearing is produced from ceramic or glass, preferably boron-silicate glass.

14. The blood pump according to one of the preceding claims, ***characterized in that*** at least part of the housing is produced from a polyether ketone.

15. A blood pump in particular according to one of the preceding claims, ***characterized in that*** the priming volume of the blood pump is below 17 ml, preferably below 15 ml.

## Revendications

1. Pompe à sang (1) comprenant un rotor (13) qui est disposé dans un carter de pompe (2) via un palier (14), sachant que le carter de pompe (2) présente une broche (10) métallique de sa paroi extérieure (5) vers l'intérieur du carter (8), ***caractérisée en ce que*** la broche (10) est conique, est disposée dans une formation du carter (9) conique et est disposée de telle façon sur un côté extérieur de la paroi extérieure (5) du carter de pompe (2) qu'elle permet à de la chaleur d'être déviée vers l'extérieur du carter.

2. Pompe à sang selon la revendication 1, ***caractérisée en ce que*** la formation du carter (9) conique s'étend à l'intérieur des aubes (15) et de préférence sur toute la longueur des aubes (15).

3. Pompe à sang selon la revendication 1 ou 2, ***caractérisée en ce que*** le diamètre du rotor est inférieur à 30 mm, de préférence inférieur à 28 mm.

4. Pompe à sang selon l'une des revendications précédentes, ***caractérisée en ce que*** le diamètre d'une plaque de couverture du rotor (19) est inférieur à 32 mm, de préférence inférieur à 30 mm.

5. Pompe à sang selon l'une des revendications précédentes, ***caractérisée en ce que*** le rotor présente un poids inférieur à 10 g, de préférence même inférieur à 8,5 g.

6. Pompe à sang selon l'une des revendications précédentes, ***caractérisée en ce que*** le carter présente une paroi de carter (5) extérieure radialement et une réception de moteur (6) s'étendant à l'intérieur de celle-ci, sachant que la réception de moteur (6) est reliée en complémentarité de forme avec la paroi de carter (5) extérieure radialement par une fermeture (7) et qu'une fente annulaire (8) est formée entre la paroi de carter (5) extérieure radialement et la réception de moteur (6).

7. Pompe à sang selon l'une des revendications précédentes, ***caractérisée en ce que*** le rotor (13) présente au moins un éléctroaimant d'accouplement (16) pour transmettre un couple de rotation sans contact d'un moteur au rotor (13).

8. Pompe à sang selon l'une des revendications précédentes, ***caractérisée en ce que*** le rotor (13) présente un perçage de circulation (17) qui conduit jusqu'au palier (14).

9. Pompe à sang selon l'une des revendications précédentes, ***caractérisée en ce que*** le rotor (13) est disposé dans le carter de pompe (2) via un palier (14) avec un segment à bille reposant dans une coquille de palier.

10. Pompe à sang selon la revendication 9, ***caractérisée en ce que*** le rotor présente le segment à bille.

11. Pompe à sang selon l'une des revendications précédentes, ***caractérisée en ce* qu'**une partie du palier est fabriquée en PTFE.

12. Pompe à sang selon l'une des revendications précédentes, ***caractérisée en ce* qu'**une partie du palier est fabriquée en acier inoxydable.

13. Pompe à sang selon l'une des revendications précédentes, ***caractérisée en ce* qu'**une partie du palier est fabriquée en céramique ou en verre, de préférence en verre borosilicaté.

14. Pompe à sang selon l'une des revendications précédentes, *caractérisée en ce qu*'au moins une partie du carter est fabriquée en polyéthercétone.

15. Pompe à sang selon l'une des revendications précédentes, ***caractérisée en ce que*** le volume d'amorçage de la pompe à sang est inférieur à 17 ml, de préférence inférieur à 15 ml.
